# EUROPÄISCHE PATENTANMELDUNG

(11) **EP 3 189 819 A1**
(43) Veröffentlichungstag der Anmeldung: **12.07.2017**
(21) Anmeldenummer: 16205202.1
(22) Anmeldetag: 20.12.2016
(51) Int. Cl.: A61F 6/14, A61M 31/00

(54) **MEDIKAMENTENABGABE-VORRICHTUNG UND EINE MEDIKAMENTENABGABE-BAUGRUPPE**

(30) Priorität: 11.01.2016 DE 102016200211
(71) Anmelder: Raumedic AG, 95213 Münchberg (DE)
(72) Erfinder: Erhard, Dominik, 95326 Kulmbach (DE)
(74) Vertreter: Rau, Schneck & Hübner Patentanwälte Rechtsanwälte PartGmbB

(57) **Zusammenfassung**

Die Erfindung betrifft eine Medikamentenabgabe-Vorrichtung zur Abgabe eines Medikaments innerhalb eines Lebewesens mit einem Gehäuse (2), mit einer innerhalb des Gehäuses (2) angeordneten Medikamenten-Speichereinheit (3) zum Speichern eines Medikaments, wobei die Medikamenten-Speichereinheit (3) zumindest bereichsweise eine flexible Wand (4) aufweist, mit mindestens einer in einer Außenwandung (8) des Gehäuses (2) angeordneten Medikamenten-Abgabeöffnung (16) zur Abgabe des Medikaments (5) und mit einem mit der flexiblen Wand (4) der Medikamenten-Speichereinheit (3) in Kontakt stehendes Quelleelement (6) zum Herausdrücken einer Menge des Medikaments (5) aus der Medikamenten-Speichereinheit (3), wobei ein mit dem Quellelement (6) in Fluidverbindung stehendes Fluid-Speicherelement (12) vorgesehen ist.

## Beschreibung

Der Inhalt der deutschen Patentanmeldung DE 10 2016 200 211.2 wird durch Bezugnahme hierin aufgenommen.

Die Erfindung betrifft eine Medikamentenabgabe-Vorrichtung sowie eine Medikamentenabgabe-Baugruppe.

Medikamentenabgabe-Vorrichtungen sind aus der EP 2 464 414 B1, WO 2012/129 497 A2 und EP 2 624 907 B1 bekannt. Die DE 697 23 589 T2 offenbart ein osmotisches Verabreichungssystem und eine Methode zur Leistungserhöhung und Verbesserung der Initialwirkung von osmotischen Verabreichungssystemen. Die DE 103 00 896 A1 beschreibt eine automatische, von Hydrogelen getriebene Fördereinrichtung mit einstellbarer Abgabecharakteristik zum Fördern eines Mediums, insbesondere Insulin. Die GB 2 347 081 A beschreibt eine Einrichtung zur Verabreichung einer förderlichen Substanz an ein Tier. Die US 5,869,078 beschreibt eine implantierbare Medikamentenabgabevorrichtung.

Eine Aufgabe der vorliegenden Erfindung besteht darin, eine Medikamentenabgabe-Vorrichtung bereitzustellen, die eine Freisetzung eines Medikaments über die gesamte Anwendungsdauer gewährleistet.

Diese Aufgabe wird mit einer Medikamentenabgabe-Vorrichtung mit den Merkmalen gemäß Anspruch 1 gelöst.

Erfindungsgemäß wurde erkannt, ein Quellelement vorzusehen, das mit einer flexiblen Wand einer Medikamenten-Speichereinheit in Kontakt steht. Durch Aufnahme einer Menge eines Fluids expandiert das Quellelement, wodurch die Medikamenten-Speichereinheit komprimiert wird. Infolgedessen wird eine Menge eines Medikaments aus der Medikamenten-Speichereinheit herausgedrückt. Als Fluid-Speicherelement kommt eine interne oder externe Komponente der Medikamentenabgabe-Vorrichtung zum Einsatz. Das Fluid-Speicherelement kann zum Speichern von Wasser oder vollentsalztem Wasser vorgesehen sein.

Vorteilhafterweise besteht das Gehäuse aus einem biokompatiblen Material, insbesondere einem Silikon.

Der in dieser Anmeldung verwendete Begriff Medikament ist nicht einschränkend zu verstehen. Der Begriff Medikament schließt sowohl eine einzelne medizinische Substanz als auch eine Medikamentenzusammensetzung ein.

Vorzugsweise liegt das Medikament in Form eines Pulvers, insbesondere in Form eines Gels, insbesondere in Form einer Flüssigkeit vor.

Die Medikamentenabgabe-Vorrichtung kann als temporäres Implantat ausgestaltet sein. Die Medikamentenabgabe-Vorrichtung kann insgesamt zur Einnahme durch das Lebewesen, also insbesondere zur oralen Einnahme, vorgesehen sein.

Bei einer Medikamentenabgabe-Vorrichtung nach Anspruch 2 ist innerhalb des Gehäuses eine Elektronik-Einheit vorgesehen. Dies führt einer kompakten Vorrichtung.

Bei einer Medikamentenabgabe-Vorrichtung nach Anspruch 3 ist ein zwischen dem Quellelement und dem Fluid-Speicherelement angeordnetes Ventil vorgesehen. Vorteilhafterweise wird hierdurch eine Steuerung einer vom Fluid-Speicherelement abzugebende Fluidmenge ermöglicht.

Bei einer Medikamentenabgabe-Vorrichtung nach Anspruch 4 steht das Ventil in Signalverbindung mit der Elektronik-Einheit. Vorteilhafterweise wird hierdurch ein Öffnen und Schließen des Ventils ermöglicht.

Bei einer Medikamentenabgabe-Vorrichtung nach Anspruch 5 ist das Quellelement 1 ein Fluid-Absorber. Hierdurch wird eine konstruktiv einfache und kostengünstige Medikamentenabgabe-Vorrichtung geschaffen.

Bei einer Medikamenten-Vorrichtung nach Anspruch 6 ist das Quellelement 1 ein Fluid-Superabsorber.

Vorteilhafterweise besteht der Fluid-Superabsorber aus einem Copolymer, beispielsweise aus Acrylsäure und Natriumacrylat. Vorteilhafterweise liegt das Copolymer als Granulat vor.

Vorteilhafterweise entsteht im Fluid-Superabsorber ein hoher osmotischer Druck, sodass auf eine aktive Förderung des Fluids, beispielsweise durch eine Pumpe oder eines Wasserstoffgenerators, verzichtet werden kann.

In einer vorteilhaften Ausführungsform ist der granulatförmige Fluid-Superabsorber in einer Membran angeordnet. Vorteilhafterweise handelt es sich hierbei um eine flexible Membran.

Bei einer Medikamentenabgabe-Vorrichtung nach Anspruch 7 weist die Elektronik-Einheit mindestens eine der nachfolgenden Komponenten auf:
- mindestens einen Sensor zur Messung eines Körperparameters eines Lebewesens,
- ein Steuer-Element zur Steuerung eines Ventils,
- ein Datenübertragungselement zur Übertragung von Daten auf ein externes Gerät und/oder
- ein Datenspeicherelement zur Speicherung von Daten.

Vorteilhafterweise ist das Datenübertragungselement zur drahtlosen Übertragung von Daten, insbesondere der von den Sensoren gemessenen Körperparametern des Lebewesens, geeignet.

Vorteilhafterweise ist der Elektronik-Einheit ein Energiespeicherelement zur Speicherung elektrischer Energie zugeordnet, wobei das Energiespeicherelement elektrische Energie für die Komponenten der Elektronik-Einheit sowie für das Öffnen und Schließen des Ventils bereitstellt.

Bei einer Medikamentenabgabe-Vorrichtung nach Anspruch 8 ist mindestens ein Sensor zur Messung eines ph-Werts einer Körperflüssigkeit, einer Körper-Temperatur, eines Blutwerts, insbesondere des Glukosewertes, oder dergleichen vorgesehen.

Bei einer Medikamentenabgabe-Vorrichtung nach Anspruch 9 ist das Fluid-Speicherelement innerhalb des Gehäuses angeordnet.

Bei einer Medikamentenabgabe-Vorrichtung nach Anspruch 10 ist das Fluid-Speicherelement außerhalb des Gehäuses angeordnet.

Eine weitere Aufgabe der Erfindung besteht darin, eine Medikamentenabgabe-Baugruppe bereitzustellen, die zur zumindest vorübergehenden Platzierung einer Medikamentenabgabe-Vorrichtung in einem Lebewesen geeignet ist.

Diese Aufgabe mit einer Medikamentenabgabe-Baugruppe mit den Merkmalen gemäß Anspruch 11 gelöst.

Es ist zweckmäßig, ein Außengehäuse mit mindestens einer weiteren Medikamenten-Abgabeöffnung vorzusehen, wobei in dem Außengehäuse eine Medikamentenabgabe-Vorrichtung angeordnet werden kann.

Vorteilhafterweise besteht das Außengehäuse aus einem biokompatiblen Material, insbesondere einem Silikon.

Bei einer Medikamentenabgabe-Baugruppe nach Anspruch 12 ist die mindestens eine Medikamentenabgabe-Öffnung der Medikamentenabgabe-Vorrichtung und die mindestens eine weitere Medikamenten-Abgabeöffnung der Medikamenten-Baugruppe zumindest teilweise überlappend angeordnet.

Bei einer Medikamenten-Baugruppe nach Anspruch 13 ist die Medikamentenabgabe-Vorrichtung mittels eines Klebers im Außengehäuse der Medikamentenabgabe-Baugruppe fixiert. Vorteilhafterweise ist der Kleber dichtend ausgeführt.

Je nach Anwendung der Medikamentenabgabe-Baugruppe kann es zweckmäßig sein, dass Fluid-Speicherelement außerhalb der Medikamentenabgabe-Vorrichtung und innerhalb des Außengehäuses der Medikamentenabgabe-Baugruppe nach Anspruch 14 anzuordnen oder das Fluid-Speicherelement außerhalb der Medikamentenabgabe-Vorrichtung und außerhalb des Außengehäuses der Medikamentenabgabe-Baugruppe nach Anspruch 15 anzuordnen.

Vorteilhafterweise ist bei einer Medikamentenabgabe-Baugruppe nach Anspruch 15 das Fluid-Speicherelement mit einer Klebeverbindung dichtend mit der Medikamentenabgabe-Baugruppe verbunden.

Ein Ausführungsbeispiel kann anhand der Figuren 1 bis 3 näher erläutert werden. In dieser zeigen
- Fig. 1: eine schematische Darstellung der Medikamentenabgabe-Vorrichtung im Ausgangszustand,
- Fig. 2: eine schematische Darstellung der Medikamentenabgabe-Vorrichtung im expandierenden Zustand des Quellelements,
- Fig. 3: eine schematische Darstellung der Medikamentenabgabe-Baugruppe.

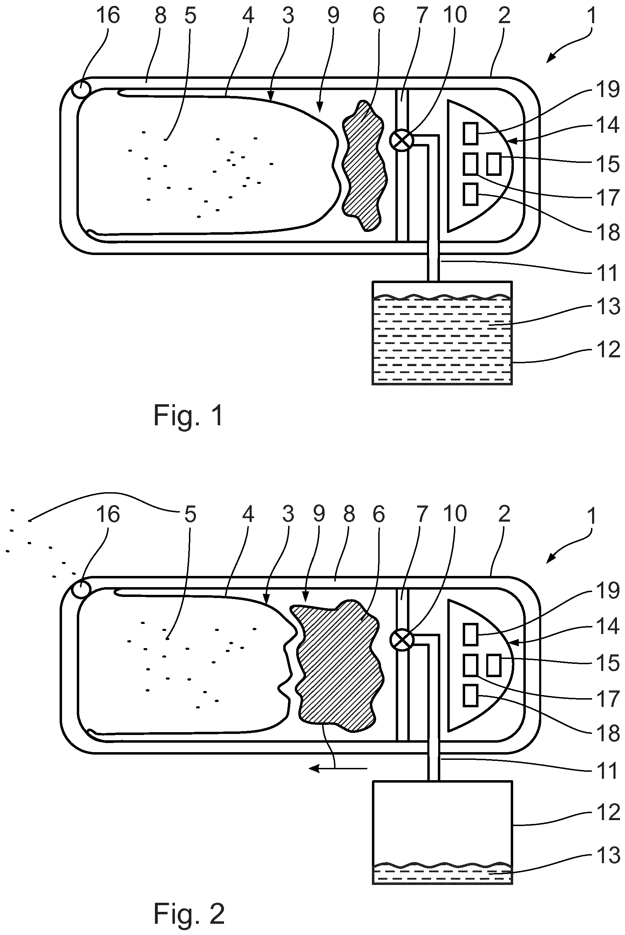

Die Fig. 1 zeigt eine Medikamentenabgabe-Vorrichtung 1 mit einem Gehäuse 2. Einer innerhalb des Gehäuses 2 angeordneten Medikamenten-Speichereinheit 3. Die Medikamenten-Speichereinheit 3 weist zumindest bereichsweise eine flexible Wand 4 zur Aufnahme eines Medikaments 5 auf. Die flexible Wand 4 steht in Kontakt mit einem Quellelement 6. Eine Gehäuseinnenwand 7 und eine Gehäuseaußenwandung 8 bilden eine Gehäusekammer 9, in der die Medikamenten-Speichereinheit 3 und das Quellelement 6 angeordnet sind. Hierbei ist das Quellelement 6 zwischen der flexiblen Wand 4 und der Gehäuseinnenwand 7 angeordnet. In der Gehäuseinnenwand 7 ist ein Ventil 10 angeordnet, das über eine Förderleitung 11 mit einem Fluid-Speicherelement 12 verbunden ist.

Das Fluid-Speicherelement 12 ist mit einem Fluid 13, insbesondere Wasser, insbesondere vollentsalztes Wasser, befüllt. In dem gezeigten Ausführungsbeispiel ist das Fluid-Speicherelement 12 außerhalb des Gehäuses 2 angeordnet. Je nach Anwendung der Medikamentenabgabe-Vorrichtung kann das Fluid-Speicherelement 12 auch innerhalb des Gehäuses 2 angeordnet sein.

In einem von der Gehäuseinnenwand 7 gegenüber dem Quellelement 6 abgetrennten Bereich ist eine Elektronik-Einheit 14 angeordnet. Die Elektronik-Einheit 14 weist ein Steuerelement 15 auf, das in Signalverbindung mit dem Ventil 10 steht. Das Steuerelement 15 dient zum Öffnen und Schließen des Ventils 10.

Beim Öffnen des Ventils 10 gelangt das Fluid 13 aufgrund des osmotischen Drucks des Quellelements 7 in die Gehäusekammer 9, sodass das Fluid 13 von dem Quellelement 6 aufgenommen wird. Durch Aufnahme des Fluids 13 expandiert das Quellelement 6, wodurch die Medikamenten-Speichereinheit 3 komprimiert wird, sodass das Medikament 5 über eine Medikamenten-Abgabeöffnung 16 abgegeben wird.

Vorzugsweise ist das Quellelement 6 ein Fluid-Absorber, insbesondere ein Fluid-Superabsorber. Der Fluid-Superabsorber ist ein Copolymer, insbesondere eine Kombination aus einer Acrylsäure und einem Natriumacrylat. Vorzugsweise liegt der Fluid-Superabsorber im trockenen Zustand als Granulat vor. Beim Öffnen des Ventils 10 gelangt das Fluid 13 in die Gehäusekammer 9, sodass dieses von dem granulatförmigen Fluid-Superabsorber aufgenommen wird und infolge der Fluidaufnahme expandiert und ein Hydrogel ausbildet. Aufgrund der Expansion des Fluid-Superabsorbers wird die flexible Wand 4 der Medikamenten-Speichereinheit 3 komprimiert. Hierdurch wird das Medikament 5 aus der Medikamenten-Speichereinheit 3 herausgedrückt und über die Medikamenten-Abgabeöffnung 16 in das Lebewesen abgegeben.

Das Steuerelement 15 kann mit weiteren Komponenten der Elektronik-Einheit 14 in Signalverbindung stehen. Eines dieser Komponenten kann ein Sensor 17 zur Messung eines Körperparameters des Lebewesens, ein Datenübertragungselement 18 zur Übertragung von Daten auf ein externes Gerät und/oder ein Datenspeicherelement 19 zur Speicherung von Daten sein. Allerdings ist diese Aufzählung nicht abschließend, es können weitere Komponenten vorgesehen sein, die dazu geeignet sind, eine steuerbare Medikamentenabgabe zu ermöglichen. Dies soll später anhand eines konkreten Beispiels näher erläutert werden.

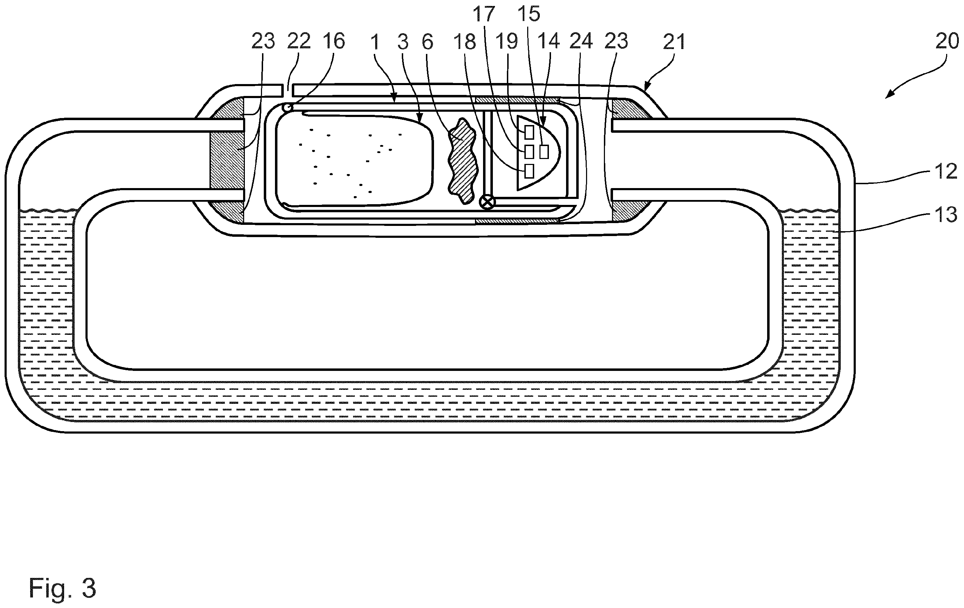

Eine Medikamentenabgabe-Baugruppe 20 gemäß Fig. 3 zur zumindest vorübergehenden Platzierung einer Medikamentenabgabe-Vorrichtung gemäß den Fig. 1 und 2. Die Medikamenten-Baugruppe 20 besteht aus einem Außengehäuse 21 mit mindestens einer weiteren Medikamenten-Abgabeöffnung 22 und eine im Außengehäuse 21 angeordnete Medikamentenabgabe-Vorrichtung 1. Weiter ist ein Fluid-Speicherelement 12 mit einem darin enthaltenen Fluid 13 vorgesehen. Das Fluid-Speicherelement 12 ist an dem Außengehäuse 20 an Klebestellen 23 dichtend fixiert. Die Medikamentenabgabe-Vorrichtung 1 ist in dem Außengehäuse 21 derart angeordnet, dass die Medikamentenabgabe-Öffnung 16 der Medikamentenabgabe-Vorrichtung 1 zumindest teilweise überlappend gegenüber der Medikamentenabgabe-Öffnung 22 des Außengehäuses 21 angeordnet ist. Die Medikamentenabgabe-Vorrichtung 1 ist mittels eines Klebers in dem Außengehäuse 21 fixiert.

Je nach Anwendung kann das Außengehäuse 21 auch das Fluid-Speicherelement umschließen.

In dem gezeigten Ausführungsbeispiel gemäß Fig. 3 ist das Fluid-Speicherelement 12 schlauchförmig ausgeführt. Dieser Schlauch ist an seinem einen Ende dichtend gegenüber der Medikamentenabgabe-Vorrichtung 1 ausgeführt, wohingegen das andere Ende des Fluid-Speicherelements 12 in Fluidverbindung mit der Medikamentenabgabe-Vorrichtung 1 steht. Hierbei ist in dem Fluid-Speicherelement 12 soviel Fluid 13 enthalten, sodass bei einer offenen Stellung des Ventils 10 das Quellelement 6 genügend Fluid 13 aufnehmen kann, um die Medikamenten-Speichereinheit 3 vollständig zu entleeren.

Eine mögliche Anwendung der Medikamentenabgabe-Baugruppe 20 ist als Intravaginalring möglich, der für mehrere Wochen in der Vagina platziert werden kann. Über die Medikamentenabgabe-Vorrichtung 1 kann dort kontinuierlich Verhütungshormone, Empfängnishormone, Insulin oder ähnliches freigesetzt werden. Je nach Anwendung der Medikamentenabgabe-Baugruppe und Ausführungsformen der Medikamentenabgabe-Vorrichtung 1 kann auch eine diskontinuierliche Abgabe eines Medikaments vorgesehen sein. Beispielsweise kann die Elektronik-Einheit 14 der Medikamentenabgabe-Vorrichtung 1 einen Sensor 17 zur Bestimmung eines Glukosewertes des Lebenswesens vorgesehen sein. Dieser misst kontinuierlich den Glukosewert. Diese gemessenen Glukosewerte können in dem Datenspeicherelement 19 gespeichert werden und über das Datenübertragungselement 18 vorteilhafterweise drahtlos an ein externes Gerät übertragen werden. Sobald ein Grenzwert überschritten wird, wird ein Signal an das Steuerelement 15 gesendet, woraufhin das Ventil 10 geöffnet wird. Durch Öffnen des Ventils 10 wird das Fluid 13 aus dem Fluid-Speicherelement 12 in die Gehäusekammer 9 abgegeben, woraufhin das Quellelement 6 expandiert und eine Menge von Insulin aus der Medikamenten-Speichereinheit 3 abgegeben wird. Beim Unterschreiten des Grenzwerts wird ein weiteres Signal an das Steuerelement 15 gesendet, woraufhin das Ventil 10 schließt. Hierdurch gelangt kein Fluid 13 mehr in die Gehäusekammer 9, woraufhin das Quellelement 6 nicht mehr expandiert und somit kein Insulin mehr abgegeben wird. Nach Erreichen der maximalen Anwendungsdauer kann die Medikamentenabgabe-Baugruppe 20 entfernt und gegebenenfalls durch eine neue ersetzt werden.

Bei einer alternativen Ausführung der Medikamentenabgabe-Vorrichtung ist diese zur insbesondere oralen Einnahme ausgestaltet. Die Medikamentenabgabe-Vorrichtung kann insgesamt in einer verschluckbaren Kapsel untergebracht sein.

## Patentansprüche

1. Medikamentenabgabe-Vorrichtung zur Abgabe eines Medikaments innerhalb eines Lebewesens
- mit einem Gehäuse (2),
- mit einer innerhalb des Gehäuses (2) angeordneten Medikamenten-Speichereinheit (3) zum Speichern eines Medikaments, wobei die Medikamenten-Speichereinheit (3) zumindest bereichsweise eine flexible Wand (4) aufweist,
- mit mindestens einer in einer Außenwandung (8) des Gehäuses (2) angeordneten Medikamenten-Abgabeöffnung (16) zur Abgabe des Medikaments (5) und
- mit einem mit der flexiblen Wand (4) der Medikamenten-Speichereinheit (3) in Kontakt stehendes Quelleelement (6) zum Herausdrücken einer Menge des Medikaments (5) aus der Medikamenten-Speichereinheit (3),
wobei
- ein mit dem Quellelement (6) in Fluidverbindung stehendes Fluid-Speicherelement (12) vorgesehen ist.

2. Medikamentenabgabe-Vorrichtung nach Anspruch 1, **gekennzeichnet durch** eine innerhalb des Gehäuses (2) angeordnete Elektronik-Einheit (14).

3. Medikamentenabgabe-Vorrichtung nach Anspruch 1 oder 2, **gekennzeichnet durch** ein zwischen dem Quellelement (6) und dem Fluid-Speicherelement (12) angeordnetes Ventil (10) zur Steuerung einer vom Fluid-Speicherelement (12) abzugebenden Fluidmenge.

4. Medikamentenabgabe-Vorrichtung nach Anspruch 2 und 3, **dadurch gekennzeichnet, dass** das Ventil (10) in Signalverbindung mit der Elektronik-Einheit (14) steht.

5. Medikamentenabgabe-Vorrichtung nach einem der Ansprüche 1 bis 4, **dadurch gekennzeichnet, dass** das Quellelement (6) ein Fluid-Absorber ist.

6. Medikamentenabgabe-Vorrichtung nach einem der Ansprüche 1 bis 5, **dadurch gekennzeichnet, dass** das Quellelement (6) ein Fluid-Superabsorber ist.

7. Medikamentenabgabe-Vorrichtung nach einem der Ansprüche 2 bis 5, **dadurch gekennzeichnet, dass** die Elektronik-Einheit (14) mindestens einer der nachfolgenden Komponenten aufweist:
- einen Sensor (17) zur Messung eines Körperparameters des Lebewesens,
- ein Steuerelement (15) zur Steuerung des Ventils (10),
- ein Datenübertragungselement (18) zur Übertragung von Daten auf ein externes
Gerät und/oder
- ein Datenspeicherelement (19) zur Speicherung von Daten ist.

8. Medikamentenabgabe-Vorrichtung nach Anspruch 7, **dadurch gekennzeichnet, dass** ein Sensor (17) zur Messung eines ph-Werts einer Körperflüssigkeit, einer Körper-Temperatur, eines Blutwerts oder dergleichen vorgesehen ist.

9. Medikamentenabgabe-Vorrichtung nach einem der Ansprüche 1 bis 8, **dadurch gekennzeichnet, dass** das Fluid-Speicherelement (12) innerhalb des Gehäuses (2) angeordnet ist.

10. Medikamentenabgabe-Vorrichtung nach einem der Ansprüche 1 bis 9, **dadurch gekennzeichnet, dass** das Fluid-Speicherelement (12) außerhalb des Gehäuses (2) angeordnet ist.

11. Medikamentenabgabe-Baugruppe zur zumindest vorübergehenden Platzierung einer Medikamentenabgabe-Vorrichtung in einem Lebewesen umfassend
- einem Außengehäuse (21) mit mindestens einer weiteren Medikamenten-Abgabeöffnung (22) und
- eine im Außengehäuse (21) angeordneter Medikamentenabgabe-Vorrichtung (1) nach einem der Ansprüche 1 bis 10.

12. Medikamentenabgabe-Baugruppe nach Anspruch 11, **dadurch gekennzeichnet, dass** die mindestens eine Medikamenten-Abgabeöffnung (16) der Medikamentenabgabe-Vorrichtung (1) und die mindestens eine weitere Medikamenten-Abgabeöffnung (22) der Medikamentenabgabe-Baugruppe (20) zumindest teilweise überlappend zueinander angeordnet sind.

13. Medikamentenabgabe-Baugruppe nach Anspruch 11 oder 12, **dadurch gekennzeichnet, dass** die Medikamentenabgabe-Vorrichtung (1) mittels eines Klebers (24) im Außengehäuse (21) der Medikamentenabgabe-Baugruppe (20) fixiert ist.

14. Medikamentenabgabe-Baugruppe nach einem der Ansprüche 11 bis 13, **dadurch gekennzeichnet, dass** das Fluid-Speicherelement (12) außerhalb der Medikamentenabgabe-Vorrichtung (1) und innerhalb des Außengehäuses (21) der Medikamentenabgabe-Baugruppe (20) angeordnet ist.

15. Medikamentenabgabe-Baugruppe nach einem der Ansprüche 11 bis 13, **dadurch gekennzeichnet, dass** das Fluid-Speicherelement (12) außerhalb der Medikamentenabgabe-Vorrichtung (1) und außerhalb des Außengehäuses (21) der Medikamentenabgabe-Baugruppe (20) angeordnet ist.
